# EUROPÄISCHE PATENTSCHRIFT

(11) **EP 0 817 766 B1**
(45) Veröffentlichungstag und Bekanntmachung des Hinweises auf die Patenterteilung: **14.04.1999**
(21) Anmeldenummer: 96906717.2
(22) Anmeldetag: 28.02.1996
(51) Int. Cl.: C07C 7/148, B01J 21/16

(54) **VERWENDUNG VON NATURSAUREN SMEKTITEN ZUR ENTFERNUNG VON OLEFINEN AUS AROMATEN ODER AROMATENGEMISCHEN**
USE OF NATURALLY ACID SMECTITES TO REMOVE OLEFINES FROM AROMATICS OR AROMATIC MIXTURES
UTILISATION DE SMECTITES ACIDES NATURELLES POUR L'ELIMINATION DES OLEFINES CONTENUS DANS DES COMPOSES AROMATIQUES OU DES MELANGES DE COMPOSES AROMATIQUES

(30) Priorität: 31.03.1995 DE 19512137
(43) Veröffentlichungstag der Anmeldung: 14.01.1998
(73) Patentinhaber: SÜDCHEMIE AG, 80333 München (DE)
(72) Erfinder: ENGELHARDT, Thomas, D-85356 Freising (DE); FLESSNER, Uwe, D-81375 München (DE); HÄHN, Reinhard, D-84186 Vilsheim (DE); ZSCHAU, Werner, D-82237 Steinebach (DE)
(74) Vertreter: Patentanwälte Dipl.-Ing. R. Splanemann Dr. B. Reitzner Dipl.-Ing. K. Baronetzky
(86) Internationale Anmeldenummer: EP9600808
(87) Internationale Veröffentlichungsnummer: WO9630321

(56) Entgegenhaltungen:
- EP-A- 0 116 469
- FR-A- 2 599 275
- CHEMICAL ABSTRACTS, vol. 111, no. 11, 11.September 1989 Columbus, Ohio, US; abstract no. 96793v, Seite 699; XP000056691 & JP,A,63 310 837 (MITSUBISHI PETROCHEMICAL CO) 19.Dezember 1988
- Fette-Seifen-Anstrichmittel,70,394-399 (1968)
- K.Tanabe, Solid Acids and Bases, Kodansha (Tokyo), 1970,23-26
- Industrial and Engineering Chemistry,41(7),1485-1489(July 1949)

## Beschreibung

Die Erfindung betrifft die Verwendung von natursauren Smektiten zur Entfernung von Olefinen aus Aromaten oder Aromatengemischen.

Die industriell bedeutendsten Aromaten Benzol, Toluol und die Xylole (BTX) werden heute nahezu ausschließlich durch katalytische oder thermische Umwandlung geeigneter Erdölfraktionen hergestellt.

Beim sogenannten katalytischen Reformieren wird eine paraffinische Naphthafraktion bei etwas 400° C mit Edelmetallbeschichteten Katalysatoren behandelt. Während dieses katalytischen Prozesses werden aus den gesättigten Kohlenwasserstoffen Aromaten gebildet. Diese Aromaten werden dann durch eine Extraktion bzw. Kristallisation von den Nichtaromaten abgetrennt und destillativ weiterverarbeitet.

Als wichtigstes Verfahren zur extraktiven Abtrennung der Benzol-Toluol-Mischungen hat sich das Sulfolan-Verfahren durchgesetzt (Ullmanns Enzyklopädie der technischen Chemie, Bd.8 (1974),S.395).

Neben den gewünschten Aromaten werden während des katalytischen Reformierung auch geringe Mengen an Olefinen gebildet. Diese Olefine, deren Gehalt in der Regel unter 1 % liegt, stören den Weiterverarbeitungsprozeß und müssen entfernt werden. Da die unerwünschten Olefine etwa die gleichen Siedepunkte aufweisen wie die Aromaten, ist eine destillative Abtrennung nicht möglich.

Als wirtschaftliches Verfahren zur Entfernung dieser Olefine hat sich weltweit die katalytische Behandlung mit Erdalkali-Aluminiumsilicaten, z.B. aktivierten Smektiten in Granulatform, durchgesetzt. Hierbei wird der Aromatenstrom bei etwa 150 - 200° C durch einen Festbettreaktor geleitet. Die Granulate wirken hierbei als Katalysator; die unerwünschten Olefine werden in höhersiedende Produkte umgewandelt, die dann destillativ leicht abgetrennt werden können.

Es ist bekannt, daß als geeignete Katalysatoren zur Entfernung der Olefine bevorzugt natürliche oder synthetische Erdalkali-Aluminiumsilicate eingesetzt werden. So sind die unterschiedlichsten Verfahren unter Verwendung von säureaktivierten Bentoniten (Bleicherden) beschrieben. In diesem Zusammenhang sei beispielsweise auf GB-1 162 945 und die DE-C-22 36 996 verwiesen. Bei den kommerziellen Produkten für die Aromatenreinigung handelt es sich in der Regel um granulierte säure-aktivierte Bentonite, wie sie zur Raffination von Nahrungsmittelölen eingesetzt werden. Die Produkte werden in der Regel in einem Korngrößenbereich zwischen 0,3 und 0,6 mm angeboten, die spezifische Oberfläche schwankt zwischen 200 und 400 m², die Ionenumtauschfähigkeit (IUF) zwischen 30 und 60 mVal/100g.

Neben den bevorzugt verwendeten säureaktivierten Bentoniten können auch synthetische Silicate, wie Al-Silicate, Mg-Silicate, Zr-Silicate, eingesetzt werden.

In der US-A 4 795 550 ist die Verwendung von Zeolithen zur Entfernung von Olefinen aus Aromatenfraktionen beschrieben. Zeolithe sind zwar sehr reaktiv; es kommt aber in ihrem engen Porensystem zur Bildung von polymeren Nebenprodukten, die zu einer sehr schnellen Desaktivierung des Katalysatorbettes führen.

Die Standzeit der säureaktivierten Bentonite in Festbett-Reaktoren schwankt je nach Prozeßbedingungen sehr stark und liegt zwischen einigen Wochen und einem Jahr. Danach ist durch Desaktivierungsprozesse so viel katalytische Aktivität verloren gegangen, daß ein Austausch des Katalysators notwendig wird. Die Prozeßbetreiber derartiger Aromatenanlagen sind daher an einem hochaktiven Katalysator interessiert, welcher sich durch eine lange Standzeit auszeichnet.

Aufgabe der vorliegenden Erfindung war es, Katalysatoren zur Entfernung von Olefinen aus Aromaten oder Aromatengemischen auf der Basis von Smektiten mit konstant hoher katalytischer Aktivität und somit langer Standzeit zu entwickeln.

Gegenstand der Erfindung ist die Verwendung von natursauren Smektiten zur Entfernung von Olefinen aus Aromaten oder Aromatengemischen. Natursaure Smektite sind an sich bekannt, wurden aber bisher nur als Bleicherden verwendet. Überraschenderweise wurde gefunden, daß sie sich auch als Katalysatoren für den vorstehend genannten Zweck eignen.

Vorzugsweise wird ein natursaurer Smektit verwendet, der folgende Eigenschaften hat:
(a) eine spezifische Oberfläche von etwa 50 bis 200 m²/g;
(b) eine Ionenumtauschfähigkeit (IUF) für austauschfähige Kationen von insgesamt etwa 40 bis 80 mVal/100 g, wobei der Anteil der Al³⁺-Ionen an der IUF etwa 5 bis 70 mVal/100 g, der Anteil der Alkali-Ionen an der IUF weniger als etwa 2,0 mVal/100 g und der Anteil der Erdalkali-Icnen an der IUF weniger als etwa 50 mVal/100 g beträgt;
(c) eine Gesamtacidität von mehr als etwa 5 mg KOH/g;
(d) einen Anteil an freien Erdalkali- oder Alkali-Ionen von weniger als 5 mVal/100g.

Der natursaure Smektit wird vorzugsweise in Form von Teilchen von mehr als etwa 0,1 mm verwendet.

Die katalytische Aktivität der erfindungsgemäß verwendeten natursauren Smektite kann durch Einbau von Al³⁺-Ionen noch weiter verbessert werden.

Somit ist Gegenstand der Erfindung auch ein Katalysator auf der Basis von natursauren Smektiten zur Entfernung von Olefinen aus Aromaten oder Aromatengemischen, der dadurch gekennzeichnet ist, daß sein Gehalt an austauschfähigen Al³⁺-Kationen um mindestens 5 mVal/100g größer ist als der des natursauren Smektits.

Der Katalysator ist vorzugsweise gekennzeichnet durch
(a) eine spezifische Oberfläche von etwa 50 bis 200 m²/g;
(b) eine Ionenumtauschfähigkeit (IUF) für austauschfähige Kationen von insgesamt etwa 40 bis 80 mVal/100 g, wobei der Anteil der Al³⁺-Ionen an der IUF etwa 10 bis 70 mVal/100 g, der Anteil der Alkali-Ionen an der IUF weniger als etwa 2,0 mVal/100 g; und der Anteil der Erdalkali-Ionen an der IUF weniger als etwa 50 mVal/100 g beträgt.
(c) eine Gesamtacidität von mehr als etwa 10 mg KOH/g;
(d) einen Anteil an freien Erdalkali- oder Alkali-Ionen von weniger als 5 mVal/100g.

Vorzugsweise beträgt der Gehalt an austauschfähigen Al³⁺-Ionen etwa 10 bis 30 mVal/100 g.

Unter "austauschfähigen Al³⁺-Ionen" versteht man Al³⁺-Ionen, die an Zwischenschichtplätzen und nicht innerhalb der Schichten des smektitischen Tonminerals gebunden sind. Sie sind im Sinne eines Ionenaustauschs chemisch gebunden, so daß sie nicht mit Wasser ausgewaschen werden können. Der Gehalt an austauschfähigen Al³⁺-Ionen wird im Rahmen der Bestimmung der Gesamt-Ionenumtauschfähigkeit (IUF) festgestellt. Hierbei wird eine Probe des Katalysators (2 g) eine Stunde in 100 ml einer 2-normalen NH₄Cl-Lösung unter Rückfluß gekocht und weitere 16 Stunden bei Raumtemperatur stehengelassen. Dann wird die Probe abfiltriert und chloridfrei gewaschen. Zunächst wird die Gesamt-IUF durch Bestimmung der in das Gitter eingetauschten NH₄⁺-Ionen bestimmt. Zu diesem Zweck wird der Gehalt an Ammoniumionen im NH₄ ausgetauschten Katalysator nach Kjeldahl bestimmt. Der Gehalt an austauschfähigen Al³⁺-Ionen wird im Filtrat der NH₄Cl-Kochlösung spektrophotometrisch bestimmt und in mVal je 100 g Katalysator ausgedrückt. Auf die gleiche Weise werden die Gehalte an Alkali- und Erdalkaliionen im Filtrat der NH₄Cl-Kochlösung bestimmt.

Nach der gleichen Methode werden die Gesamt-IUF und der Anteil der austauschfähigen Kationen der Vergleichssubstanzen bestimmt.

Der erfindungsgemäße Katalysator kann zusätzlich zu den austauschfähigen Al³⁺-Ionen freie Al³⁺-Ionen auf der Oberfläche, in den Poren und im Zwischenkornvolumen enthalten. Diese Al³⁺-Ionen können durch einfaches Auswaschen des Katalysators mit Wasser entfernt werden.

Zu diesem Zweck wird eine Probe des Katalysators (10 g) vor dem Kochen mit der NH₄Cl-Lösung 5-mal mit je 100 ml Wasser von 25°C in einer Filternutsche gewaschen, bis im letzten Waschwasseranteil kein Aluminium mehr nachzuweisen ist. Das Aluminium wird in den vereinigten Waschwässern wiederum spektrophotometrisch bestimmt. Auf die gleiche Weise können auch die anderen freien Kationen bestimmt werden.

Die Bestimmung der IUF und des Anteils der einzelnen Ionen an der Gesamt-IUF sowie die Bestimmung der freien Kationen kann auch bei den smektitischen Vergleichsmaterialien vorgenommen werden. Hierbei wird durch das Ergebnis ein zu hoher Gehalt an austauschfähigen Calcium- oder Magnesiumionen vorgetäuscht. Dies ist dadurch bedingt, daß das smektitische Ausgangsmaterial häufig mit Calcium- und/oder Magnesiumcarbonat verunreinigt ist, die beim Kochen mit der NH₄Cl-Lösung gelöst werden.

Man nimmt an, daß sowohl die austauschfähigen Al³⁺-Ionen als auch die freien Al³⁺-Ionen eine katalytische Wirkung nach Art der Lewis-Säuren ausüben. Bisher wurden Lewis-Säuren (insbesondere Aluminiumchlorid) nur in der homogenen Katalyse eingesetzt. Es ist daher überraschend, daß auch die austauschfähig gebundenen Al³⁺-Ionen in den erfindungsgemäßen heterogenen Katalysatoren nach Art von Lewis-Säuren wirken. Besonders überraschend ist, daß der katalytische Effekt der austauschfähigen Al³⁺-Kationen stärker ist als der katalytische Effekt der freien Al³⁺-Ionen.

Gegenstand der Erfindung ist ferner ein Verfahren zur Herstellung eines Katalysators, das dadurch gekennzeichnet ist, daß man ein natursaures smektitisches Ausgangsmaterial mit einer Aluminiumsalzlösung behandelt, deren Menge so bemessen ist, daß sich der Gehalt an austauschfähigen Al³⁺-Ionen auf mindestens 10 mVal/100 g erhöht und daß gegebenenfalls noch ein Anteil an freien Al³⁺-Ionen vorliegt.

Hierbei geht man im allgemeinen von einem natursauren smektitischen Ausgangsmaterial mit einem Gehalt an austauschfähigen Al³⁺-Ionen von weniger als 5 mVal/100 g aus, obgleich man auch von einem Material mit einem höheren Gehalt an austauschfähigen Al³⁺-Ionen ausgehen kann. Die katalytische Aktivität eines derartigen Ausgangsmaterials, das bereits die Bedingungen für einen erfindungsgemäßen Katalysator erfüllt, kann durch den Eintausch von weiteren Al³⁺-Ionen noch gesteigert werden. Die erfindungsgemäße Behandlung kann also in zwei oder mehreren Stufen durchgeführt werden.

Bei der ersten bevorzugten Variante des erfindungsgemäßen Verfahrens geht man so vor, daß man das Aluminiumsalz in einem 1-bis 5-fachen molaren Überschuß, bezogen auf die IUF, verwendet, einen Eintausch der Al³⁺-Ionen gegen ein- und zweiwertige Kationen vornimmt und den Katalysator anschließend wäscht, um freie, d.h. wasserlösliche Aluminiumsalze sowie Salze der ein- und zweiwertigen Kationen zu entfernen, falls diese im Gesamtprozeß stören.

Hierbei wird eine Suspension des natursauren smektitischen Tonminerals (Bleicherdesuspension) mit einer Aluminiumsalzlösung, insbesondere mit einer Aluminiumsufatlösung, imprägniert, wobei ein direkter Ionenaustausch stattfindet. Nach mehrstündigem Austausch bei Raumtemperatur oder erhöhter Temperatur wird der Überschuß an Aluminiumionen ausgewaschen, worauf das ausgetauschte Bleicherdeprodukt anschließend getrocknet wird. Hieran schließt sich der Granulationsprozeß an.

Nach der zweiten Variante kann man ein zuvor klassiertes Granulat eines natursauren Bentonits (Bleicherdegranulat) mit überschüssiger wäßriger Aluminiumsulfatlösung einsprühen, wobei ein Teil der Al³⁺-Ionen austauschfähig gebunden wird und ein anderer Teil der Al³⁺-Ionen in freier Form, d.h. lediglich adsorbiert vorliegt. Nach dem Einsprühen wird das Material bei etwa 80 bis 150°C, vorzugsweise bei etwa 100°C getrocknet.

Für eine erheblich gesteigerte katalytische Aktivität sind bei beiden Varianten einige Prozent Aluminiumsalz ausreichend. Grundsätzlich sind verschiedene Aluminiumsalze geeignet. Aus kommerziellen und technischen Gründen ist Aluminiumsulfat dem Aluminiumchlorid vorzuziehen.

Nach beiden Varianten kann man den mit Al³⁺-Ionen angereicherten Katalysator in ein Granulat mit einer Teilchengröße von > 0,1 mm überführen.

Gegenstand der Erfindung ist ferner die Verwendung des erfindungsgemäßen Katalysators zur Entfernung von Olefinen aus Aromaten oder Aromatengemischen.

Die in den Ansprüchen und in der Beschreibung angebenen weiteren Merkmale werden wir folgt bestimmt:
1. Spezifische Oberfläche: nach der BET-Methode (Einpunktmethode mit Stickstoff nach DIN 66131, wobei die Probe zuvor 10 Stunden bei 150°C entgast wird).
2. Gesamtacidität Eine Probe des Katalysators (5 g) wird in 250 ml Wasser bei 95°C suspendiert und anschließend filtriert; das erhaltene klare Filtrat wird mit 0,1 n KOH-Lösung gegen Phenolphthalein titriert. Die Gesamtacidität wird in mg KOH/100 g Katalysator ausgedrückt.

Die Erfindung ist durch die nachstehenden Beispiel in nicht einschränkender Weise erläutert.

### Vergleichsbeispiel 1

### Herstellung eines Granulats aus unbehandetem Bentontit

100 kg eines getrockneten bayerischen Calciumbentonit (Tonsil® 13 der Fa. Süd-Chemie AG) mit den in Tabelle I angegebenen Eigenschaften werden gemahlen und im Drais-Intensivmischer mit 32 kg Wasser 10 Minuten gemischt und anschließend auf einem Einschneckenextruder zu 2 mm dicken Strängen verpreßt.

Die feuchten Stränge werden bei 110° C 10 Stunden getrocknet und anschließend in einem Walzenbrecher mit einem Spaltabstand von 1 mm zerkleinert. Anschließend wird die Fraktion zwischen 0,3 mm und 0,6 mm abgesiebt.

### Vergleichsbeispiel 2

### Herstellung eines unmodifizierten Bleicherdegranulats

100 kg einer Bleicherde in Form eines salzsauer aktivierten Bentonits (Tonsil® Optimum FF der Fa. Süd-Chemie AG) mit den in Tabelle 1 angegebenen Eigenschaften werden im Drais-Intensivmischer mit 45 kg Wasser 10 Minuten gemischt und anschließend auf einem Einschneckenextruder zu 2 mm dicken Strängen verpreßt.

Die feuchten Stränge werden wie nach Vergleichsbeispiel 1 weiterbehandelt.

### Beispiel 1

### Herstellung eines Granulats auf Basis eines natursauren Rohtons

100 kg eines natursauren vermahlenen und getrockneten Rohtons mit den in Tabelle I angegebenen Eigenschaften werden im Drais-Intensivmischer mit 35 kg Wasser 10 Minuten gemischt und anschließend auf einem Einschneckenextruder zu 2 mm dicken Strängen verpreßt.

Die feuchten Stränge werden wie nach Vergleichsbeispiel 1 weiterbehandelt.

### Beispiel 2

### Eintausch von austauschfähigen Al³⁺-Ionen in natursauren Bentonit

100 kg des natursauren Bentonits von Beipiel 1, werden in 1000 Liter destilliertem Wasser unter starkem Rühren dispergiert. Nach einer Rührzeit von 3 Stunden werden 7,5 kg Aluminiumsulfat in fester Form zugegeben und anschliessend bei Raumtemperatur weitere 12 Stunden gerührt.

Die aluminiumsulfat-haltige Suspension wird anschließend auf einer Filterpresse entwässert und mit 1000 Liter demineralisiertem Wasser nachgewaschen. Der feuchte Filterkuchen wird 12 Stunden bei 120° C getrocknet.

Dann wird der getrocknete Filterkuchen auf einem Brecher zerkleinert und die Fraktion zwischen 0,3 und 0,6 mm abgesiebt. Die Eigenschaften des so erhaltenen Katalysators sind in Tabelle I angegeben.

### Beispiel 3

### Eintausch von austauschfähigen und freien Al³⁺-Ionen in natursauren Bentonit.

100 kg des Granulats von Beispiel 1 werden in einen Pelletisierteller gebracht und anschließend mit 20 Liter einer 25-%igen Aluminiumsulfatlösung besprüht. Das imprägnierte Granulat wird 24 Stunden bei 120°C getrocknet.

Die Al³⁺-Ionen sind zum Teil in das Gitter eingetauscht; zum Teil befinden sie sich auf der Oberfläche des Materials. Die Eigenschaften des imprägnierten Granulats sind in Tabelle I angegeben.

### Anwendungsbeispiel

### (Bestimmung der katalytischen Aktivität der Katalysatoren bei der Olefinentfernung aus Aromatengemischen)

Ein aus der Sulfolanextraktion erhaltenes Aromatengemisch (70 Gew.-% Benzol, 30 Gew.-% Toluol) mit einem Olefinanteil entsprechen einem Bromindex von 50 mg Br₂/100 g (nach ASTM D1491) wird mit einer HPLC-Pumpe über eine HPLC-Säule geleitet, in der sich das zu untersuchende Katalysator als Schüttung befindet (Reaktorvolumen 10 ml). Die HPLC-Säule befindet sich in einem regelbaren Thermostat, in dem die Temperatur bei 200° C konstant gehalten wird. Um die Gasbildung bei diesen hohen Temperaturen zu vermeiden, befindet sich zwischen HPLC-Säule und den elektronisch gesteuerten Probenahmenventilen ein Rückdruckregler. An diesem Rückdruckregler wird ein Gegendruck von 50 bar eingestellt.

Mit Hilfe der HPLC-Pumpe wird eine LHSV (liquid hourly space velocity = Flüssig-Raumgeschwindigkeit) von 12 h⁻¹ eingestellt. Über die zeitgesteuerten Probenahmeventile wird alle 24 Stunden eine Probe der gereinigten Aromatenmischung entnommen und der Bromindex bestimmt.

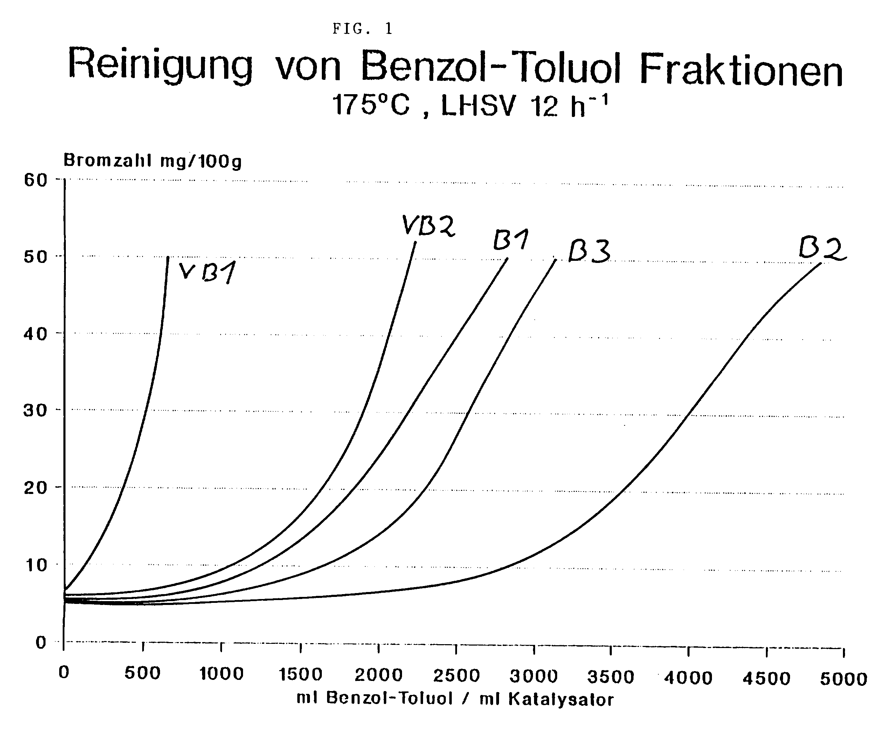

Zur Beurteilung der katalytischen Aktivität wird der Bromindex gegen die Betriebsdauer (Tage) aufgetragen. Die Ergebnisse sind in der beigefügten Figur angegeben.

Man erkennt, daß der säureaktivierte Bentonit nach Vergleichsbeispiel 2 eine längere Standzeit als der unbehandelte Bentonit nach Vergleichsbeispiel 1 hat. Die längste Standzeit hat jedoch der Katalysator nach Beispiel 2, der nur austauschfähige Al³⁺-Kationen enthält. Eine etwas geringere Standzeit haben der natursaure Katalysator nach Beispiel 1 und der mit austauschfähigen und freien Al³⁺-Ionen angereicherte Katalysator nach Beispiel 3. Daraus ergibt sich, daß der erfindungsgemäße Effekt dann am ausgeprägtesten ist, wenn der Katalysator nur durch austauschfähig gebundene Al³⁺-Ionen angereichert ist.

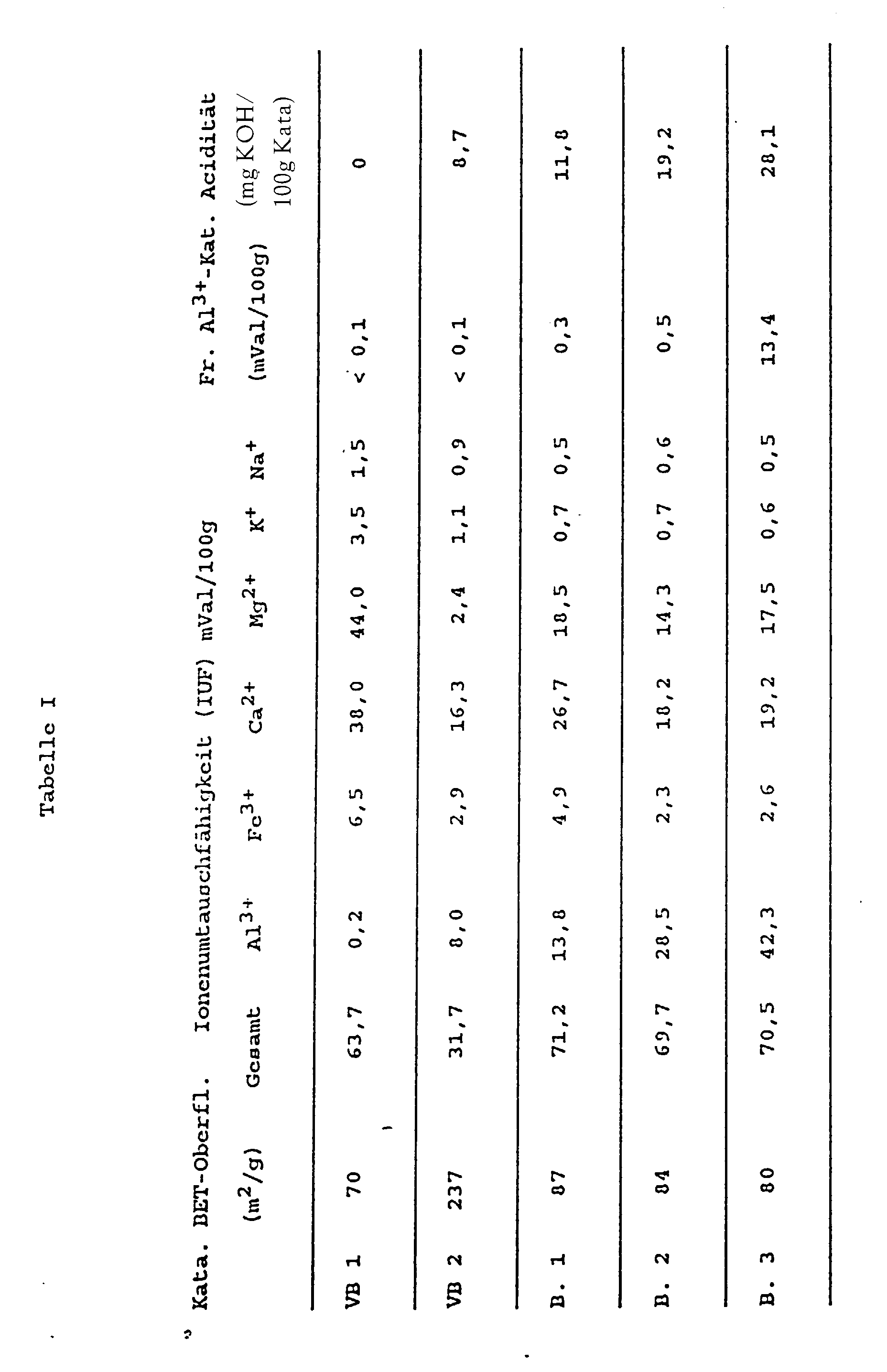

## Patentansprüche

1. Verwendung von natursauren Smektiten zur Entfernung von Olefinen aus Aromaten oder Aromatengemischen.

2. Verwendung nach Anspruch 1, dadurch gekennzeichnet, daß der natursaure Smektit folgende Eigenschaften hat:
(a) eine spezifische Oberfläche von etwa 50 bis 200 m²/g;
(b) eine Ionenumtauschfähigkeit (IUF) für austauschfähige Kationen von insgesamt etwa 40 bis 80 mVal/100 g, wobei der Anteil der Al³⁺-Ionen an der IUF etwa 5 bis 70 mVal/100 g, der Anteil der Alkali-Ionen an der IUF weniger als etwa 2,0 mVal/100 g und der Anteil der Erdalkali-Ionen an der IUF weniger als etwa 50 mVal/100 g beträgt;
(c) eine Gesamtacidität von mehr als etwa 5 mg KOH/g;
(d) einen Anteil an freien Erdalkali- oder Alkali-Ionen von weniger als 5 mVal/100 g.

3. Verwendung nach Anspruch 1 oder 2, dadurch gekennzeichnet, daß der natursaure Smektit in Form von Teilchen von mehr als etwa 0,1 mm vorliegt.

4. Katalysator auf der Basis von natursauren Smektiten zur Entfernung von Olefinen aus Aromaten oder Aromatengemischen, dadurch gekennzeichnet, daß sein Gehalt an austauschfähigen Al³⁺-Kationen um mindestens 5 mVal/100g größer ist als der des natursauren Smektits.

5. Katalysator nach Anspruch 4, gekennzeichnet durch
(a) eine spezifische Oberfläche von etwa 50 bis 200 m²/g;
(b) eine Ionenumtauschfähigkeit (IUF) für austauschfähige Kationen von insgesamt etwa 40 bis 80 mVal/100 g, wobei der Anteil der Al³⁺-Ionen an der IUF etwa 10 bis 70 mVal/100 g, der Anteil der Alkali-Ionen an der IUF weniger als etwa 2,0 mVal/100 g; und der Anteil der Erdalkali-Ionen an der IUF weniger als etwa 50 mVal/100 g beträgt.
(c) eine Gesamtacidität von mehr als etwa 10 mg KOH/g;
(d) einen Anteil an freien Erdalkali- oder Alkali-Ionen von weniger als 5 mVal/100 g.

6. Katalysator nach Anspruch 4 oder 5, dadurch gekennzeichnet, daß der Gehalt an austauschfähigen Al³⁺-Ionen etwa 10 bis 30 mVal/100 g beträgt.

7. Katalysator nach einem der Ansprüche 4 bis 6, dadurch gekennzeichnet, daß er zusätzlich zu den austauschfähigen Al³⁺-Ionen freie Al³⁺-Ionen auf der Oberfläche, in den Poren und im Zwischenkornvolumen enthält.

8. Katalysator nach einem der Ansprüche 4 bis 7, dadurch gekennzeichnet, daß er in Form von Teilchen mit einer Größe von mehr als etwa 0,1 mm vorliegt.

9. Verfahren zur Herstellung eines Katalysators nach einem der Anprüche 4 bis 8, dadurch gekennzeichnet, daß man ein natursaures smektitisches Ausgangsmaterial mit einer Aluminiumsalzlösung behandelt, deren Menge so bemessen ist, daß sich der Gehalt an austauschfähigen Al³⁺-Ionen auf mindestens 10 mVal/100 g erhöht und daß gegebenenfalls noch ein Anteil an freien Al³⁺-Ionen vorliegt.

10. Verfahren zur Herstllung des Katalysators nach Anspruch 4, 5, 6 oder 8, dadurch gekennzeichnet, daß man das Aluminiumsalz in einem 1- bis 5-fachen molaren Überschuß verwendet, einen Eintausch der Al³⁺-Ionen gegen ein- und zweiwertige Kationen vornimmt und den Katalysator anschließend wäscht.

11. Verfahren nach Anspruch 9 oder 10, dadurch gekennzeichnet, daß man den mit Al³⁺-Ionen angereicherten Katalysator in ein Granulat mit einer Teilchengröße von > 0,1 mm überführt.

12. Verwendung des Katalysators nach einem der Ansprüche 4 bis 8, bzw. hergestellt nach einem der Ansprüche 9 bis 11, zur Entfernung von Olefinen aus Aromaten oder Aromatengemischen.

## Claims

1. Use of naturally acid smectites to remove olefins from aromatic compounds or aromatic mixtures.

2. Use according to Claim 1, characterised in that the naturally acid smectite has the following properties:
(a) a specific surface area of approximately 50 to 200 m²/g;
(b) an ion exchange capacity (IEC) for exchangeable cations of, in total, approximately 40 to 80 meq/100 g, wherein the proportion of Al³⁺ ions in terms of the IEC is approximately 5 to 70 meq/100 g, the proportion of alkali ions in terms of the IEC is less than approximately 2.0 meq/100 g and the proportion of alkaline earth ions is less than approximately 50 meq/100 g;
(c) a total acidity of more than approximately 5 mg KOH/g;
(d) a proportion of free alkaline earth or alkali ions less than 5 meq/100 g.

3. Use according to Claim 1 or 2, characterised in that the naturally acid smectite is present in the form of particles larger than approximately 0.1 mm.

4. A catalyst based on naturally acid smectites for removing olefins from aromatic compounds or aromatic mixtures, characterised in that its content of exchangeable Al³⁺ cations is at least 5 meq/100 g greater than that of naturally acid smectite.

5. A catalyst according to Claim 4, characterised by
(a) a specific surface area of approximately 50 to 200 m²/g;
(b) an ion exchange capacity (IEC) for exchangeable cations of, in total, approximately 40 to 80 meq/100 g, wherein the proportion of Al³⁺ ions in terms of the IEC is approximately 10 to 70 meq/100 g, the proportion of alkali ions in terms of the IEC is less than approximately 2.0 meq/100 g, and the proportion of alkaline earth ions in terms of the IEC is less than approximately 50 meq/100 g;
(c) a total acidity of more than approximately 10 mg KOH/g;
(d) a proportion of free alkaline earth or alkali ions less than 5 meq/100 g.

6. A catalyst according to Claim 4 or 5, characterised in that the content of exchangeable Al³⁺ ions is approximately 10 to 30 meq/100 g.

7. A catalyst according to any one of Claims 4 to 6, characterised in that it contains free Al³⁺ ions on the surface, in the pores and in the intergranular volume, in addition to the exchangeable Al³⁺ ions.

8. A catalyst according to any one of the Claims 4 to 7, characterised in that it is present in the form of particles of a size larger than approximately 0.1 mm.

9. A process for the preparation of a catalyst according to any one of the Claims 4 to 8, characterised in that a naturally acid smectitic starting material is treated with an aluminium salt solution, the amount of which is calculated so that the content of exchangeable Al³⁺ ions is increased to at least 10 meq/100 g and that optionally a proportion of free Al³⁺ ions is still present.

10. A process for the preparation of the catalyst according to Claim 4, 5, 6 or 8, characterised in that the aluminium salt is used in a one to five-fold molar excess, an exchange of Al³⁺ ions is carried out for monovalent and divalent cations and the catalyst is then washed.

11. A process according to Claim 9 or 10, characterised in that the catalyst enriched with the Al³⁺ ions is converted to a granular material with a particle size of > 0.1 mm.

12. Use of the catalyst according to any one of Claims 4 to 8 or prepared according to any one of Claims 9 to 11 for removing olefins from aromatic compounds or aromatic mixtures.

## Revendications

1. Utilisation de smectites acides naturelles pour éliminer des oléfines à partir de composés aromatiques ou de mélanges de composés aromatiques.

2. Utilisation selon la revendication 1, caractérisée en ce que la smectite acide naturelle a les propriétés suivantes :
(a) une aire spécifique d'environ 50 à 200 m²/g ;
(b) une capacité d'échange d'ions (CEI), portant sur les cations échangeables, égale en tout à environ 40 à 80 mVal/100 g, la contribution des ions Al³⁺ à la CEI étant d'environ 5 à 70 mVal/100 g, la contribution des ions de métaux alcalins à la CEI étant inférieure à environ 2,0 mVal/100 g, et la contribution des ions de métaux alcalino-terreux à la CEI étant inférieure à environ 50 mVal/100 g ;
(c) une acidité totale supérieure à environ 5 mg KOH/g
(d) une proportion d'ions libres de métaux alcalins ou alcalino-terreux inférieure à 5 mVal/100 g.

3. Utilisation selon la revendication 1 ou 2, caractérisée en ce que la smectite acide naturelle se présente sous forme de particules ayant une granulométrie supérieure à environ 0,1 mm.

4. Catalyseur à base de smectites acides naturelles pour éliminer des oléfines à partir de substances aromatiques ou de mélange de substances aromatiques, caractérisé en ce que sa teneur en cations Al³⁺ échangeables est d'au moins 5 mVal/100 g supérieure à celle de la smectite acide naturelle.

5. Catalyseur selon la revendication 4, caractérisé par :
(a) une aire spécifique d'environ 50 à 200 m²/g ;
(b) une capacité d'échange d'ions (CEI), portant sur les cations échangeables, égale en tout à environ 40 à 80 mVal/100 g, la contribution des ions Al³⁺ à la CEI étant d'environ 10 à 70 mVal/100 g, la contribution des ions de métaux alcalins à la CEI étant inférieure à environ 2,0 mVal/100 g, et la contribution des ions de métaux alcalino-terreux à la CEI étant inférieure à environ 50 mval/100 g ;
(c) une acidité totale supérieure à environ 10 mg KOH/g ;
(d) une proportion d'ions libres de métaux alcalins ou alcalino-terreux inférieure à 5 mVal/100 g.

6. Catalyseur selon la revendication 4 ou 5, caractérisé en ce que la teneur en ions Al³⁺ échangeables est d'environ 10 à 30 mval/100 g.

7. Catalyseur selon l'une des revendications 4 à 6, caractérisé en qu'il contient, en plus des ions Al³⁺ échangeables, des ions Al³⁺ libres sur la surface, dans les pores et dans le volume intergranulaire.

8. Catalyseur selon l'une des revendications 4 à 7, caractérisé en ce qu'il se présente sous forme de particules ayant une granulométrie supérieure à environ 0,1 mm.

9. Procédé de préparation d'un catalyseur selon l'une des revendications 4 à 8, caractérisé en ce qu'on traite une smectite acide naturelle de départ avec une solution d'un sel d'aluminium dont la quantité est définie de façon que la teneur en ions Al³⁺ échangeables augmente d'au moins 10 mVal/100 g, et que l'on ait éventuellement encore une certaine proportion d'ions Al³⁺ libres.

10. Procédé de préparation du catalyseur selon la revendication 4, 5, 6, ou 8, caractérisé en ce qu'on utilise le sel d'aluminium selon un excès 1 à 5 fois molaire, on remplace les ions Al³⁺ par des cations monovalents et divalents, puis on lave le catalyseur.

11. Procédé selon la revendication 9 ou 10, caractérisé en ce qu'on convertit le catalyseur enrichi en ions Al³⁺ en un granulat ayant une granulométrie supérieure à 0,1 mm.

12. Utilisation du catalyseur selon l'une des revendications 4 à 8, ou préparé selon l'une des revendications 9 à 11, pour éliminer des oléfines à partir de substances aromatiques ou de mélange de substances aromatiques.
